(19) Europäisches Patentamt
European Patent Office
Office européen des brevets

(11) Publication number: **0 121 719 B1**

# (12) EUROPEAN PATENT SPECIFICATION

(45) Date of publication of the patent specification: **25.06.86**

(51) Int. Cl.$^4$: **C 12 Q 1/00**

(21) Application number: **84102027.4**

(22) Date of filing: **27.02.84**

(54) **Process for determining enzymatic activity.**

(30) Priority: **02.03.83 JP 32857/83**

(43) Date of publication of application: **17.10.84 Bulletin 84/42**

(45) Publication of the grant of the patent: **25.06.86 Bulletin 86/26**

(84) Designated Contracting States: **DE FR GB IT**

(56) References cited:
**GB - A - 1 486 474**
**US - A - 4 218 535**

(73) Proprietor: **Oriental Yeast Co., Ltd., 10, 6-ban, 3-chome, Azusawa Itabashi-ku, Tokyo (JP)**

(72) Inventor: **Yamauti, Juniti, 3-44-7, Hara-machi, Suita-shi Osaka-fu (JP)**
Inventor: **Takagahara, Isamu, 5-7-13, Yamato-Higashi, Kawanishi-shi Osaka-fu (JP)**
Inventor: **Fujii, Katsumi, 3-21, Yamada-Nishi, Suita-shi Osaka-fu (JP)**
Inventor: **Yamashita, Jinpei, 3-12-2, Hiyoshidai, Takatsuki-shi Osaka-fu (JP)**
Inventor: **Horio, Takekazu, 2-7, Oohata-cho, Takatsuki-shi Osaka-fu (JP)**

(74) Representative: **Thomsen, Dieter, Dr.rer.nat., Kaiser-Ludwig-Platz 6, D-8000 München 2 (DE)**

EP 0 121 719 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

*Detailed Description of the Invention:*

This invention relates to a process for determining the activity of an object enzyme by using a conjugate enzyme which has a relatively large Michaelis constant ($K_m$) in a chain reaction system.

More particularly it relates to a process for calculating the activity of an object enzyme which comprises using a conjugate enzyme which has a relatively large Michaelis constant ($K_m$) in a considerably smaller amount than sufficient to thereby determine $V_2$ in a chain reaction system.

In chain enzyme reactions, there are generally at least two enzymes which play a conjugative role to form a chain reaction system. For example, adenosine deaminase and glutamate dehydrogenase form a conjugated enzymatic system in the following chain reaction scheme (12):

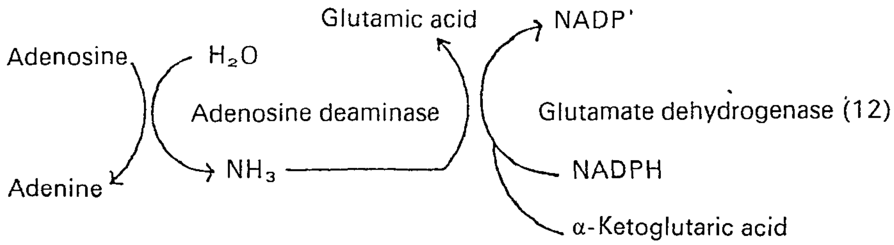


In the present invention, an enzyme of which the activity is to be determined in such a chain reaction system will be called the object enzyme, e.g. adenosine deaminase in the above-mentioned chain reaction scheme, while the other the conjugate enzyme, e.g., glutamate dehydrogenase in this example.

There are numerous chain reaction systems other than that described above with adenosine deaminase and glutamate dehydrogenase which usually occurs in vivo. For example, it is also possible to couple hexokinase with glucose-6-phosphate dehydrogenase to form a chain reaction system.

The following enzymes are also capable of coupling with glutamate dehydrogenase (GIDH) similarly to adenosine deaminase to form a chain reaction system respectively by the reaction as described below:

| | |
|---|---|
| AMP deaminase | (EC 3,5,4,6) |
| Adenine deaminase | (EC 3,5,4,2) |
| Guanine deaminase | (EC 3,5,4,3) |
| Arginine deaminase | (EC 3,5,3,6) |
| Urease | (EC 3,5,1,5) |

In the same way, Acetylcholine esterase (AchE) can be measured with acetate kinase (AK) which has a high $K_m$.

$$\text{Acetylcholine} + H_2O \xrightarrow{\text{AchE}} \text{Choline} + \text{acetate}$$

$$\text{Acetate} + \text{ATP} \xrightarrow{\text{AK}} \text{Acetylphosphate} + \text{ADP}$$

$$\text{ADP} + \text{PEP} \xrightarrow{\text{PK}} \text{Pyruvate} + \text{ATP}$$

$$\text{Pyruvate} + \text{NADH} \xrightarrow{\text{LDH}} \text{Lactate} + \text{NAD}^+$$

This invention provides a process which is effective in determining the activity of an object enzyme by using a conjugate enzyme in a chain reaction system which has a relatively large Michaelis constant (e.g. $K_m > 1$ mM).

It is generally very difficult to determine the activity of an object enzyme by using an enzyme which serves as a conjugate enzyme in a chain reaction system and has a relatively large Michaelis constant as well. Therefore a sufficient amount of the conjugate enzyme must be added in a conventional process, although troubles might often occur in such a case in that cost, solubility or purity of the conjugate enzyme would make it impossible to add a sufficient amount thereof.

Heretofore no other means than adding a seemingly sufficient amount of a conjugate enzyme are employed in a conventional process to prepare a diagnostic determining kit by using a chain reaction system involving a conjugate enzyme having a relatively large Michaelis constant.

Needless to say, it is necessary to add a considerably large amount of a conjugate enzyme if it has a relatively large Michaelis constant.

A process for determining the activity of an object enzyme has been known in which the object enzyme is subjected to a chain reaction with one or more conjugate enzyme(s) and the determined reaction velocity of the final conjugate enzyme is regarded as the activity of the object enzyme. However, this determining process is effective only under such conditions that the conjugate enzyme has a small Michaelis constant and is present in a sufficient amount. That is to say, this process has such disadvantages that a considerably higher enzyme concentration will be necessary with an increase in the Michaelis constant and the reaction velocity of the conjugate enzyme will not approach to that of the object enzyme during the determination.

As the result of our researches for determining the activity of an object enzyme by using a small amount of a conjugate enzyme, we have succeeded in calculating the reaction velocity of the object enzyme by determining the maximum reaction velocity of a conjugate enzyme accurately, calculating $r_2$ by equation (6) and determining the reaction velocity of the conjugate enzyme ($V_2$), or by diluting the conjugate enzyme to prepare at least two solutions of different concentrations, determining $V_2$ at each concentration of $E_2$ and determining the activity ratio $r_2$ by plotting.

Thus this invention provides a process for determining the activity of an object enzyme in a chain

reaction system consisting of an object enzyme and a conjugate enzyme, which comprises determining the maximum reaction velocity of the conjugate enzyme ($V_{max;2}$) and dividing the reaction velocity of the conjugate enzyme ($V_2$) by the reaction velocity ratio ($r_2$; reaction velocity of conjugate enzyme/reaction velocity of object enzyme) which is determined by substituting ($V_{max;2}$) in the following equation (6),

$$V_{max;2} = \frac{F(n_2, r_2) \times K_{m;2}}{t} + V_1 \qquad (6)$$

or diluting the conjugate enzyme to prepare at least two solutions of different concentrations (nothing but the concentration ratio is known), determining $V_2$ at each concentration of $E_2$ and dividing the resulting $V_2$ by $r_2$ determined by plotting.

According to the process of the present invention for determining the activity of an object enzyme by using a small amount of a conjugate enzyme, it is possible to prepare an excellent diagnostic determining kit economically and efficiently.

*Brief Description of the Drawings:*

Fig. 1 shows a relationship among $r_2$, $n_2$ and $F_2$. Fig. 2 shows a relationship between $r_{2b}/r_{2a}$ ($= V_{2b}/V_{2a}$) and $r_{2a}$, in which A, B, C and D represent a case where $V_1 \cdot t/2 \times K_{m;2}$ equals to 0, 0.01, 0.02 and 0.05 respectively. Fig. 3 shows curves which respectively indicate a relationship between $V_{2b}/V_{2a}$ and $r_{2a}$ as $V_{max;2b}/V_{max;2a}$ is changed, wherein $(1-k) \times V_1 \times t/K_{m;2} = 0$. Fig. 4 shows an example of adenosine deaminase determination under activity-controlled conditions, in which $V_1$ represents the reaction velocity of ADA determined 1 min and 10 sec to 1 min and 50 sec after the initiation of the reaction by using a sufficient amount of GIDH (17,000 μM/min), $V_1/2$ represents an activity equal to one-half $V_1$, X represents $V_2$ determined 10 min and 10 sec to 10 min and 50 sec after the initiation of the reaction, by using 235 μM/min of GIDH activity and Y represents $V_2$ determined 10 min and 10 sec to 10 min and 50 sec after the initiation of the reaction, by using 94 μM/min of GIDH activity.

*Background of the Invention:*

In a binary chain reaction system scheme (1), we shall generally assume that 1) the reaction velocity (activity) $V_1$ of an object enzyme $E_1$ is constant; and 2) the reaction of a conjugate enzyme $E_2$ obeys Michaelis-Menten equation.

$$S_1 \xrightarrow[V_1]{E_1} S_2 \xrightarrow[V_2]{E_2} P \qquad (1)$$

where

S: substrate

P: final product

E: enzyme

V: velocity

The above-mentioned scheme (1) leads to the following equation (2), which in turn leads to equation (3).

$$d[S_2]/dt = V_1 - V_{max;2}/(1 + K_{m;2}/[S_2]) \qquad (2)$$

$$t = -\{K_{m;2}/(V_1 - V_{max;2})\}\{V_2/(V_2 - V_{max;2})\} + \{V_{max;2}/(V_1 - V_{max;2})\}\ell n \{V_{max;2}(V_2 - V_1)/V_1(V_2 - V_{max;2})\} \qquad (3)$$

where

$V_1$: reaction velocity of $E_1$ (M/min)

$V_2$: reaction velocity of $E_2$ (M/min)

$V_{max;2}$: maximum reaction velocity of $E_2$ (M/min)

$K_{m;2}$: Michaelis constant (M) of $E_2$ with regard to $S_2$

t: time (min)

Equation (3) is transformed into equation (4) by defining $V_{max;2}/V_1 = n_2$ and $V_2/V_1 = r_2$.

$$t = \frac{K_{m;2}}{V_1 - V_{max;2}}\left\{\frac{r_2}{r_2 - n_2} + \frac{n_2}{1 - n_2}\ell n \frac{n_2(r_2 - 1)}{r_2 - n_2}\right\} \qquad (4)$$

Then equation (5) is defined as follows and substituted into equation (4) thereby to lead to equation (6).

$$\frac{r_2}{r_2 - n_2} + \frac{n_2}{1 - n_2}\ell n \frac{n_2(r_2 - 1)}{r_2 - n_2} = F(n_2, r_2) \qquad (5)$$

$$V_{max;2} = \frac{F(n_2, r_2) \times K_{m;2}}{t} + V_1 \qquad (6)$$

$F(n_2, r_2)$ represents a function of $n_2$ and $r_2$. Two separate reaction systems in which $n_2$ and $r_2$ are identical respectively have the same $F_2$. Fig. 1 shows a relationship among $r_2$, $n_2$ and $F_2$, indicating a relationship between $n_2$ and $F_2$ at various $r_2$ from 0.1 to 0.999, i.e. $V_2/V_1 = 0.10$-$0.999$. If $r_2$ is constant, $F_2$ is substantially constant when $n_2 > 5$. In other words, when $n_2$ is greater than 5 and $V_{max;2}$ is known, it is possible to determine $r_2$. Then it is also possible to determine $V_1$ by dividing the determined $V_2$ by $r_2$. This process will be referred to as Process 1.

However, it is more troublesome than expected to determine $V_{max;2}$. In addition, $V_{max;2}$ should be determined each time when $E_2$ would not be so stable. It can not be ensured that $E_2$ might not be inactivated during storage, particularly in the case of preparing a kit or the like. The following Process 2 makes it possible to determine $r_2$ when $V_{max;2}$ is not known accurately. Then it is also possible to determine $V_1$ by dividing the determined $V_2$ by the resulting $r_2$.

First, $E_2$ concentration, although unknown, is defined as $V_{max;2a}$. Then the $E_2$ is diluted accurately to a concentration of $V_{max;2b}$. The known dilution, i.e. $V_{max;2b}/V_{max;2a}$, is defined as k, and $V_2$ values determined by adding $E_2$ in an amount of $V_{max;2a}$ and $V_{max;2b}$ are defined as $V_{2a}$ and $V_{2b}$ respectively. $r_{2a}$ and $r_{2b}$ are defined as $V_{2a}/V_1$ and $V_{2b}/V_1$ respectively to define $F_2$ thereof as $F_{2a}$ and $F_{2b}$ respectively. Thus equation (6) is transformed into equations (7) and (8) and these equations lead to equation (9), which in turn leads to equation (10).

$$V_{max.2a} = F_{2a} \times K_{m.2}/t + V_1 \quad (7)$$

$$V_{max.2b} = F_{2b} \times K_{m.2}/t + V_1 \quad (8)$$

$$\frac{V_{max.2b}}{V_{max.2a}} = \frac{F_{2b} \times K_{m.2}/t + V_1}{F_{2a} \times K_{m.2}/t + V_1} = k \quad (9)$$

$$F_{2b} = k \times F_{2a} - (1-k) \times V_1 \times t/K_{m.2} \quad (10)$$

Theoretically, k may take any value within the range of 0 to 1. When k=0.5 (i.e. two-fold dilution), equation (10) may be transformed into the following equation (11).

$$F_{2b} = 0.5F_{2a} - 0.5\ V_1 \times t/K_{m.2} \quad (11)$$

When n is greater than 5, $F_{2a}$ corresponding to $r_{2a}$ of 0.1 to 0.999 may be determined by Fig. 1. Then the resulting $F_{2a}$ is substituted into equation (11) to determine $F_{2b}$, by which the corresponding $r_{2b}$ may be determined. Fig. 2 can be obtained by plotting $r_{2b}/r_{2a}$ ($=V_{2b}/V_{2a}$) against $r_{2a}$ when the second term of the right side of equation (11), i.e. $V_1\ t/2 \times K_{m.2}$, is 0, 0.01, 0.02 and 0.05. Curves A, B, C and D represent the individual case as described above. When the determination range of $V_1$ is set to 0 to 10 µM/min, it is necessary that $10 \times 10^{-6} \times t/2 \times K_{m.2} \leqq 0.01$, that is to say, $t/K_{m.2} \leqq 2 \times 10^3$ to render the above-mentioned second term smaller than 0.01. For example, when using $E_2$ having $K_{m.2} = 5 \times 10^{-3}$ M, it is necessary to determine $V_{2a}$ and $V_{2b}$ within 10 min after the initiation of the reaction, i.e. $t \leqq 10$. In this case, error of $r_{2a}$ is represented by the transverse distance between curves A and B. Accordingly it is shown by determining $r_{2a}$ by using curve E which is obtained by connecting midpoints of the transverse distances between curves A and B that a $V_{max,2}$ concentration which makes $V_{2b}/V_{2a}$ higher than 0.56, 0.58 and 0.60 respectively must be selected to render the error lower than 10%, 5% and 3% when the determination range of $V_1$ is 0 to 10 µM/min.

When $V_2$ is determined one min after the initiation of the reaction in the determination range of $V_1$ of 0 to 10 µM by using the same $E_2$ ($K_{m.2} = 5 \times 10^{-3}$ M) as described above, the second term of the right side of equation (11) approximately equals to zero and the relationship between $V_{2b}/V_{2a}$ and $r_{2a}$ is approximated by curve A. Consequently determination with little error may be carried out by using curve A. As a matter of course, a larger amount of $E_2$ (i.e. higher $V_{max,2}$) should be added in such a case than with the case in which determination is carried out 10 min after the initiation of the reaction. The second term of the right side of equation (11) will become smaller with an increase in $K_{m;2}$, resulting in a higher accuracy of the determination. When identical determination accuracy is expected, the period from the initiation of the reaction to the determination will be prolonged with an increase in $K_{m;2}$, i.e. a smaller amount of $E_2$ may be used. It is also possible to change the determination accuracy by using a proper curve in Fig. 2. For example, when $V_2$ is determined 10 min after the initiation of the reaction by using $E_2$ ($K_{m;2} = 5 \times 10^3$ M), accurate $V_1$ may be determined by using curve B if $V_1$ is approximately 10 µM/min.

As described above, Fig. 2 may be drawn depending on $E_2$ concentration ratio, $K_{m;2}$, t and desired range of $V_1$ determination. Fig. 3 is obtained by determining k as 0.1 to 0.9 and the second term of the right side as zero in equation (10).

In practice, $V_{2b}/V_{2a}$ is determined by $V_{2a}$ and $V_{2b}$ which are determined by the addition of $E_2$ of $V_{max;2a}$ and $V_{max,2b}$ respectively, and $r_2$ is determined by the curve obtained by plotting $V_{2b}/V_{2a}$ against $r_{2a}$. Activity determinations of a series of samples are carried out by using $E_2$ of $V_{max;2a}$. Then $V_1$ of each sample may be determined by dividing the resulting $V_2$ by $r_{2a}$. Process 2 as described above may be applied to a ternary chain reaction system and so on. For example, it may be considered that $V_2 \doteqdot V_3$ and $V_1 \doteqdot V_2$ respectively, when $E_3$ having small $K_m$ is added in a greater amount than that of $E_2$ having large $K_m$, or contrary to this $E_3$ having large $K_{m;3}$ is added in a smaller amount than that of $E_2$ having small $K_m$. Therefore the theory in a binary chain reaction system may be applied as such.

In the following examples are described several embodiments to illustrate the present invention.

*Example 1*

*Process 1 (a process for determining $V_1$ by an accurate determination of $V_{max;2}$)*

The activity of adenosine deaminase (ADA) was determined by using glutamate dehydrogenase (GIDH) obtained from yeast in the chain reaction system of scheme (12).

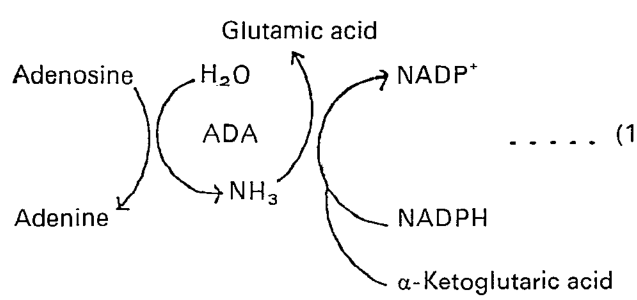


..... (12)

First, $K_m$ of GIDH with regard to ammonia, which was used as a conjugate enzyme, was determined. The reaction velocity was determined in the presence of an 80 mM potassium phosphate buffer solution (pH 7.4), 5 mM of α-ketoglutaric acid (α-KG) and 0.2 mM of NADPH while varying the concentration of ammonium acetate. Then the $K_m$ was determined to be $3.4 \times 10^{-3}$ M from a Line-

weaver-Burk plot of the determined values. Subsequently $V_{max;2}$ (in Fig. 1, F = 0.69 when n ≧ 10) which made $V_2$ to become 50% of $V_1$ when t = 10 (min) was found to be 235 μM/min (0.235 U/ml) by calculating with the resulting $K_m$ and equation (6). Then a different amount of ADA (i.e. different $V_1$) from $E_2$ of $V_{max;2}$ = 235 μM/min (determined at 30° C, pH 7.4) was added to 0.7 mM (approximately 20 times as much as $K_m$) of adenosine, 5 mM of α-KG, 0.25 mM of NADPH and a 50 mM potassium phosphate buffer solution to determine GIDH activity 10 min after the initiation of the reaction at 30° C (curve X in Fig. 1). $V_2$ was also determined under the same conditions except for reducing the amount of GIDH to 1/2.5 (94 μM/min) (curve Y in Fig. 4). A sufficient amount of GIDH (17,000 μM/min) was added to determine $V_1$ in the period of 1 min and 10 sec to 1 min and 50 sec after the initiation of the reaction (curve $V_1$ in Fig. 4). Comparing the dotted line which shows one-half $V_1$ ($V_1/2$ in Fig. 4) with curve A, it was found that ADA activity can be determined with an error less than 2% by doubling the determined activity of GIDH within a range of ADA activity of 0 to 16 μM/min. That is to say, accurate $V_1$ can be determined by dividing $V_2$ by 0.5 when $V_2$ is within a range of 0 to 8 μM/min. On the other hand, when $V_1$ =40 μM/min, the theoretical value of $V_2$ was 89% of $(V_1)/2$ while the determined value thereof was 78% of $(V_1)/2$. Further deviation of a determined $V_1$ from a theoretical one was observed when $V_1$ was still higher. Such a phenomenon seemed to result from an experimentally lower value of $V_2$ because the NADPH concentration when carrying out the determination had become too low when $V_1$ was too high, as the former had been adjusted to 0.25 mM to put the absorbance of the reaction liquor at 340 nm within the range determinable by a spectrometer in setting the determination conditions ($K_m$ of GIDH with regard to NADPH was 1.3 × $10^{-4}$ M). In a determination by using 94 μM/min of GIDH (curve B), the determinable range of $V_2$ in proportion to $V_1$ would be broadened to 0 to 30 μM/min, as a matter of course. The ratio $r_2$ of $V_2$ to $V_1$ in this case was calculated to be 0.25 theoretically, which was approximately equal to $V_1$ determined by dividing the resulting $V_2$ by 0.25.

*Example 2*

*Process 2 (a process for determining the activity ratio $r_{2a}$ (= $V_{2a}/V_1$) by diluting $E_2$ to prepare at least two solutions of different concentrations (nothing but concentration ratio is known) and determining $V_2$ at each $E_2$ concentration (determined value; $V_{2a}$, $V_{2b}$...)*

Determination was carried out in the same reaction system as in Example 1 by using 5 mM of α-KG, 0.25 mM of NADPH, a 50 mM potassium phosphate buffer solution (pH 7.4), ADA ($E_1$) and GIDH ($E_2$) solutions of various concentrations. $E_2$ solutions of two concentrations ($V_{max;2a}$ = 250 and 208 μM/min) were prepared and each was further diluted to 1/2, 2/5 and 1/3. Each $E_2$ solution was added to the reaction liquor and a decrease in NADPH concentration was observed for a period of 10 min and 10 sec to 10 min and 50 sec after the initiation of the reaction to determine $V_2$. $V_2$'s determined by using $E_2$ solution, 1/2 $E_2$ solution, 2/5 $E_2$ solution and 1/3 $E_2$ solution will be referred to as $V_{2a}$, $V_{2b1}$, $V_{2b2}$ and $V_{2b3}$ respectively. $V_1$ was determined by determining $r_{2a}$ corresponding to the activity ratio of each combination ($V_{2b1}/V_{2b}$, $V_{2b2}/V_{2a}$ and $V_{2b3}/V_{2a}$) from $V_{2b}/V_{2a}$ vs. $r_{2a}$ curve (Fig. 3) and dividing $V_{2a}$ by the resulting $r_{2a}$. Results are shown in the following table.

*Table 1*

$V_1$ = 10.0 μM/min

$V_{max;2a}$ = 250 μM/min

| $V_{max;2}$ | $V_2$ | $V_{2b}/V_{2a}$ | $r_{2a}$ | $V_1$ (= $V_{2a}/r_{2a}$) |
|---|---|---|---|---|
| $V_{max;2a}$ | $V_{2a}$ 5.02 | — | — | — |
| $V_{max;2a/2}$ | $V_{2b1}$ 2.98 | 0.594 | 0.530 | 9.47 |
| $V_{max;2a/2.5}$ | $V_{2b2}$ 2.43 | 0.485 | 0.515 | · 9.73 |
| $V_{max;2a/3}$ | $V_{2b3}$ 2.07 | 0.412 | 0.485 | 10.35 |

$V_{max;2a}$ = 208 μM/min

| $V_{max;2}$ | $V_2$ | $V_{2b}/V_{2a}$ | $r_{2a}$ | $V_1$ (= $V_{2a}/r_{2a}$) |
|---|---|---|---|---|
| $V_{max;2a}$ | $V_{2a}$ 4.42 | — | — | — |
| $V_{max;2a/2}$ | $V_{2b1}$ 2.52 | 0.569 | 0.440 | 10.04 |
| $V_{max;2a/2.5}$ | $V_{2b2}$ 2.07 | 0.468 | 0.450 | 9.85 |
| $V_{max;2a/3}$ | $V_{2b3}$ 1.73 | 0.391 | 0.430 | 10.27 |

A part from this, $V_1$ was determined by a conventional process. That is to say, a sufficient amount (17 mM/min) of $E_2$ was added to a reaction liquor. Then $V_2$ was determined one min after the initiation of the reaction and the resulting value was regarded as an approximation of $V_1$ ($V_1$ = 10.0 μM/min). The value of $V_{2a}/r_{2a}$ was approximately equal to $V_1$ (10 μM/min) in either case of $V_{max;2a}$ = 250 μM/min or 208 μM/min. These results suggest that it is possible to determine $V_1$ by calculating $V_2/r_2$ with the addition of a considerably smaller amount of a conjugate enzyme having a relatively large $K_m$ than the case of a conventional process under adequate conditions. It further suggests that the activity of an object enzyme can be determined with an appreciable accuracy by diluting the conjugate enzyme to prepare two solutions of different concentrations and determining $V_2$ at each concentration, so long as the concentration (dilution ratio) of the added conjugate enzyme was known, without the knowledge of the activity of the added conjugate enzyme accurately.

The resulting $V_1$ and the substrate concentration $K_{m;1}$ are substituted in Michaelis-Menten equation to thereby determine $V_{max;1}$, from which unit (U) of $E_1$ may be determined.

**Claim**

A process for determining the activity of an object enzyme in a chain reaction system consisting of an object enzyme and a conjugate enzyme, which comprises determining the maximum reaction velocity of the conjugate enzyme ($V_{max.2}$) and dividing the reaction velocity of the conjugate enzyme ($V_2$) by the reaction velocity ratio ($r_2$; reaction velocity of conjugate enzyme/reaction velocity of object enzyme) which is determined by substituting ($V_{max;2}$) in the following equation (6),

$$V_{max.2} = \frac{F(n_2, r_2) \times K_{m.2}}{t} + V_1 \qquad (6)$$

or diluting the conjugate enzyme to prepare at least two solutions of different concentrations (nothing but the concentration ratio is known), determining $V_2$ at each concentration and dividing the resulting $V_2$ by $r_{2a}$ determined by plotting.

**Revendication**

Procédé pour la détermination de l'activité d'une enzyme objet dans un système de réactions en chaîne consistant en une enzyme objet et une enzyme conjuguée, qui consiste à déterminer la vitesse de réaction maximale de l'enzyme conjuguée ($V_{max.2}$) et à diviser la vitesse de réaction de l'enzyme conjuguée ($V_2$) par le rapport des vitesses de réaction ($r_2$, vitesse de réaction de l'enzyme conjuguée/vitesse de réaction de l'enzyme objet) qui est déterminé en introduisant ($V_{max.2}$) dans l'équation (6) suivante:

$$V_{max.2} = \frac{F(n_2, r_2) \times K_{m;2}}{t} + V_1 \qquad (6)$$

ou bien à diluer l'enzyme conjuguée pour préparer au moins deux solutions de concentrations différentes (le rapport des concentrations est seul connu), à déterminer $V_2$ à chaque concentration et à diviser la $V_2$ résultante par $r_{2a}$ déterminé par traçage de la courbe.

**Patentanspruch**

Verfahren zum Bestimmen der Aktivität eines Objekt-Enzyms in einem Kettenreaktionssystem, bestehend aus einem Objekt-Enzym und einem Conjugat-Enzym, das das Bestimmen der maximalen Reaktionsgeschwindigkeit des Conjugat-Enzyms ($V_{max;2}$) und die Division der Reaktionsgeschwindigkeit des Conjugat-Enzyms ($V_2$) durch das Reaktionsgeschwindigkeitsverhältnis ($r_2$; Reaktionsgeschwindigkeit vom Conjugat-Enzym/Reaktions geschwindigkeit vom Objekt-Enzym) umfasst, welches durch Substituieren ($V_{max;2}$) in der folgenden Gleichung (6)

$$V_{max;2} = \frac{F(n_2, r_2) \times K_{m;2}}{t} + V_1 \qquad (6)$$

bestimmt ist, oder Verdünnen des Conjugat-Enzyms zur Herstellung von wenigstens zwei Lösungen unterschiedlicher Konzentration (nur das Konzentrationsverhältnis ist bekannt), Bestimmen von $V_2$ bei jeder Konzentration und Teilen des resultierenden $V_2$-Wertes durch $r_{2a}$, bestimmt durch graphisches Auftragen.

FIG. 1

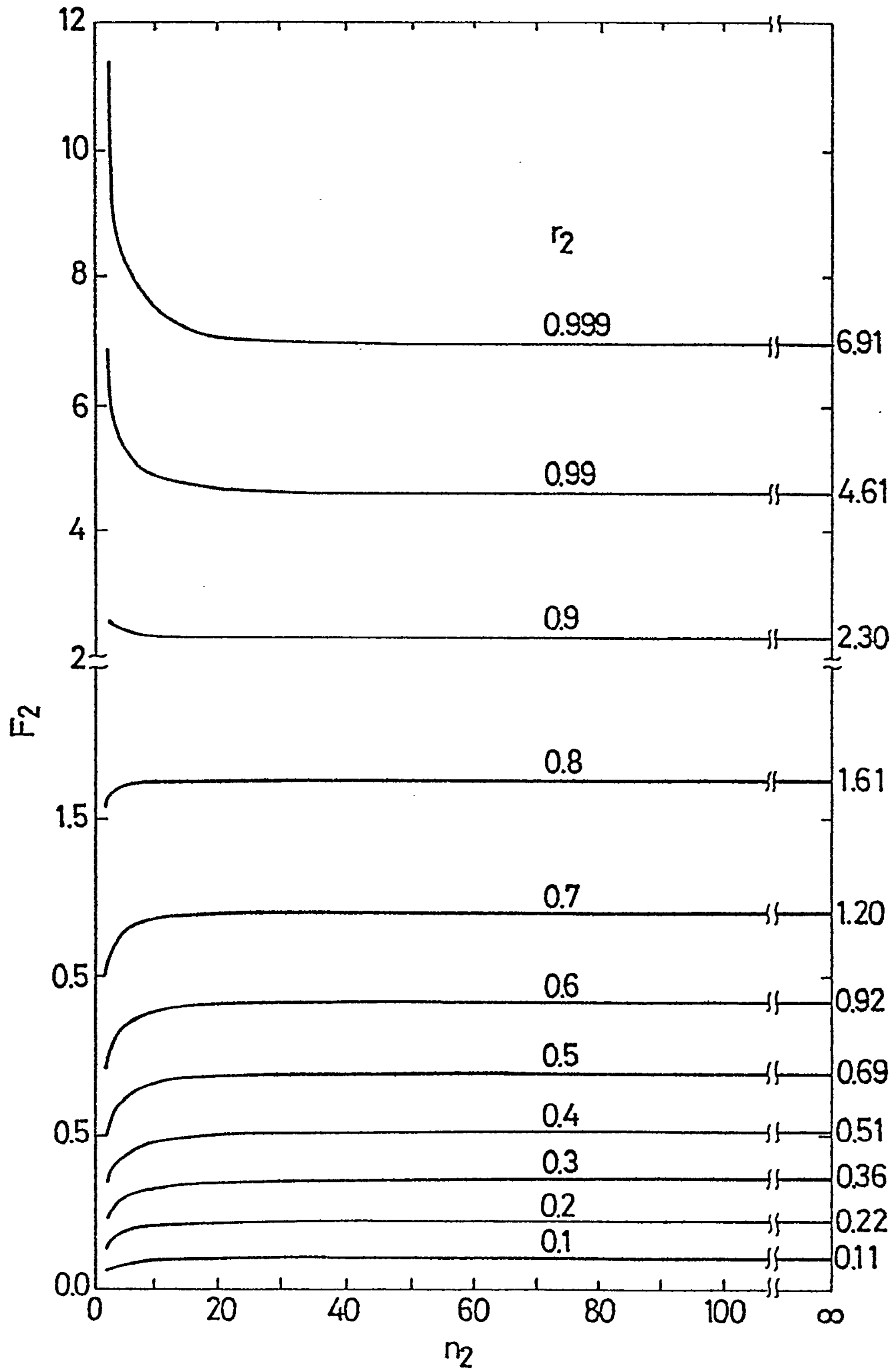
12
10
8
6
4
2
1.5
0.5
0.5
0.0
$r_2$
0.999
0.99
0.9
0.8
0.7
0.6
0.5
0.4
0.3
0.2
0.1
6.91
4.61
2.30
1.61
1.20
0.92
0.69
0.51
0.36
0.22
0.11
$F_2$
0
20
40
60
80
100
∞
$n_2$

FIG. 2

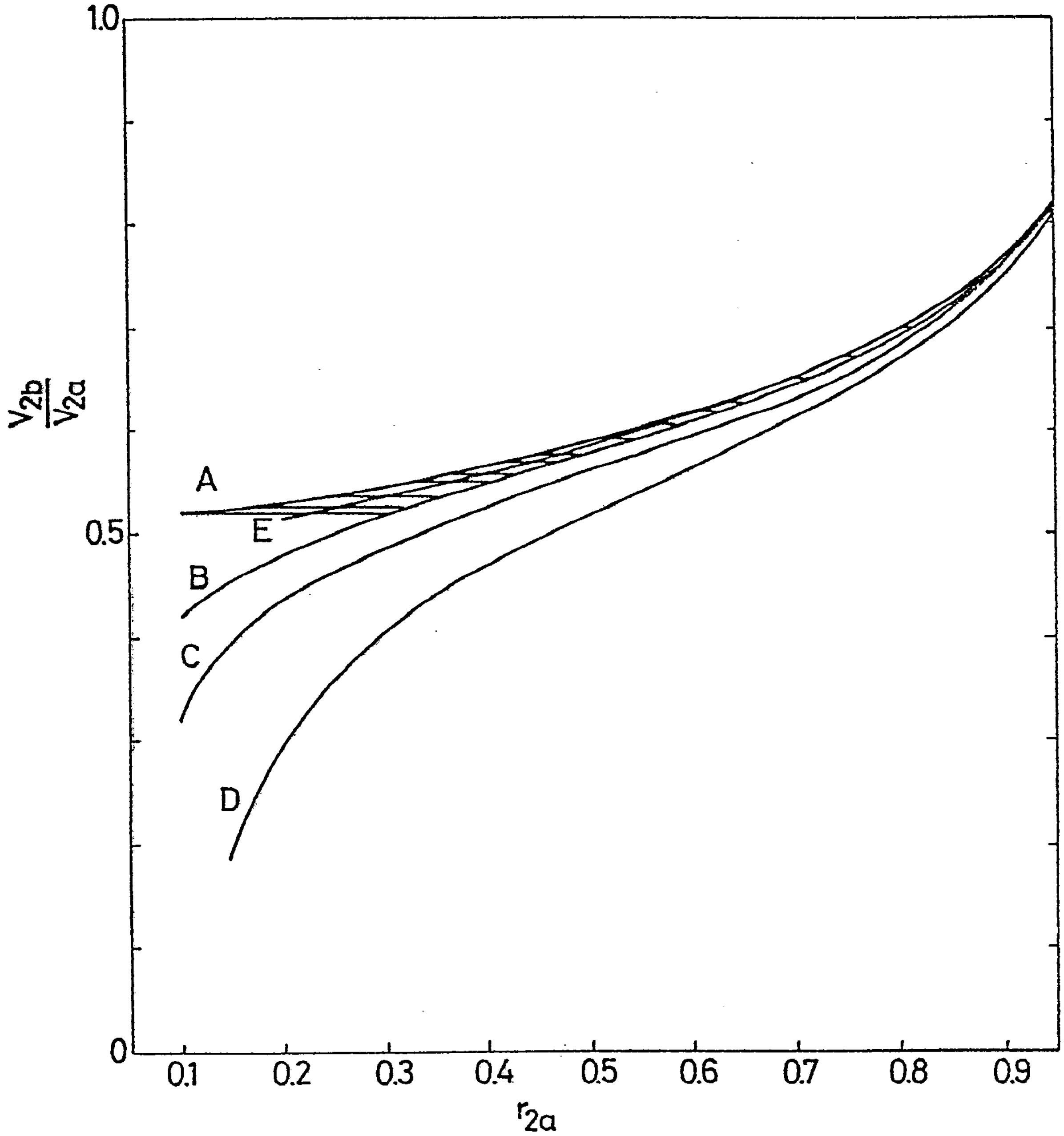

FIG. 3

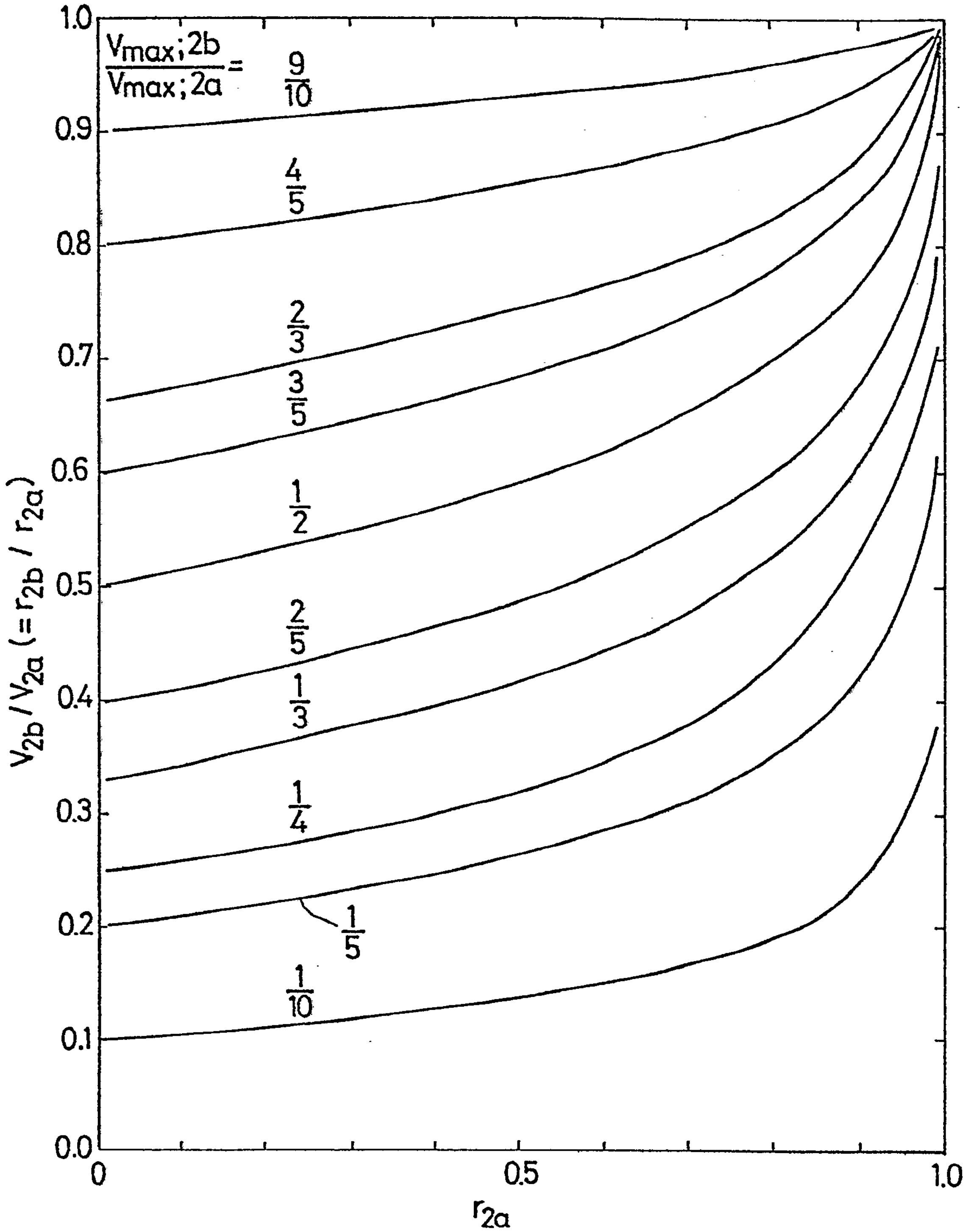
Vmax;2b / Vmax;2a =
9/10
4/5
2/3
3/5
1/2
2/5
1/3
1/4
1/5
1/10
1.0
0.9
0.8
0.7
0.6
0.5
0.4
0.3
0.2
0.1
0.0
0
0.5
1.0
V2b / V2a (= r2b / r2a)
r2a

FIG. 4

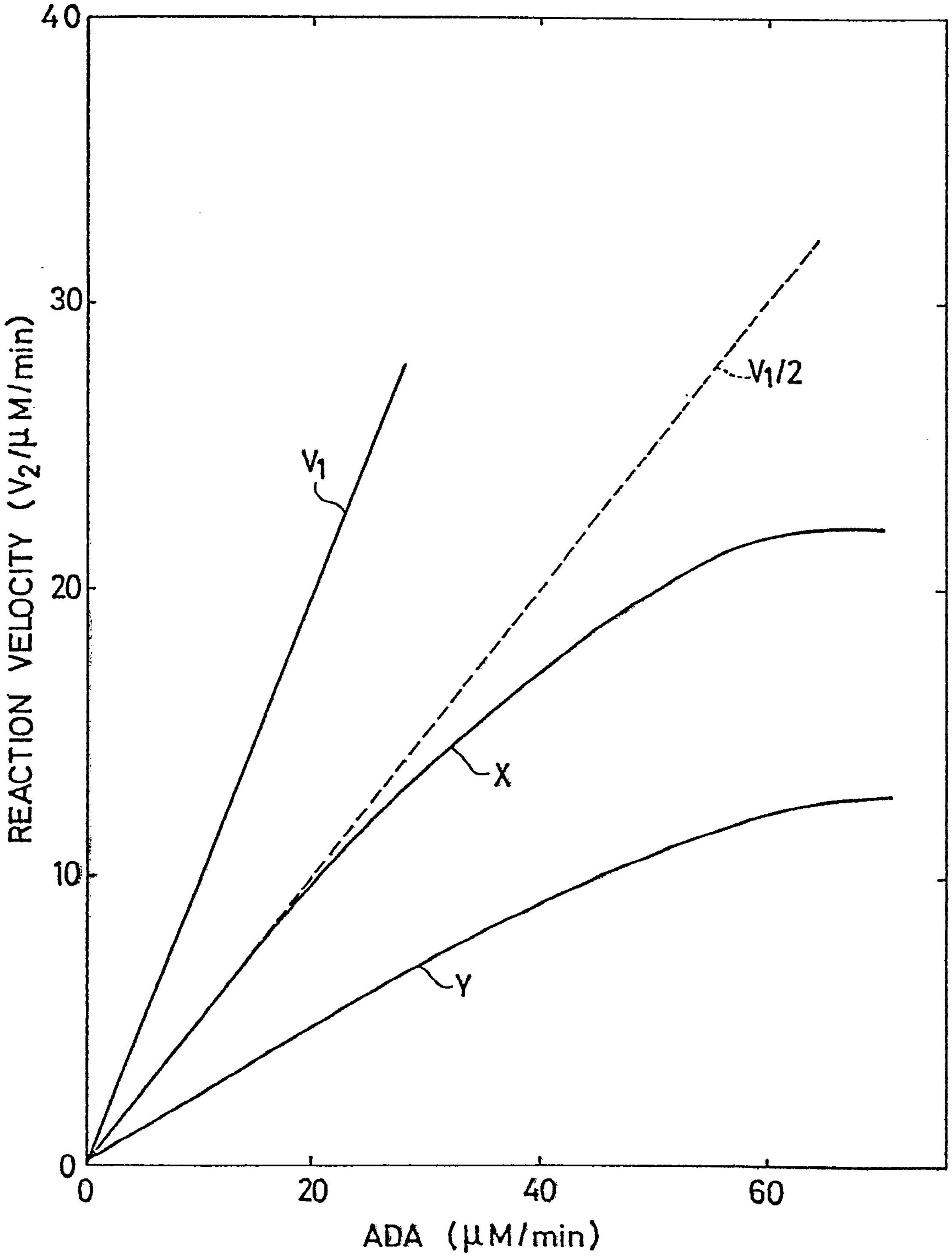
REACTION VELOCITY ($V_2$/μM/min)
ADA (μM/min)
0
10
20
30
40
0
20
40
60
$V_1$
$V_1/2$
X
Y